(19) Europäisches Patentamt
European Patent Office
Office européen des brevets

(11) Veröffentlichungsnummer: **0 371 499 A2**

(12) # EUROPÄISCHE PATENTANMELDUNG

(21) Anmeldenummer: **89122075.8**

(22) Anmeldetag: **30.11.89**

(51) Int. Cl.5: **C07D 213/803, //C07C229/30**

(30) Priorität: **01.12.88 DE 3840554**

(43) Veröffentlichungstag der Anmeldung: **06.06.90 Patentblatt 90/23**

(84) Benannte Vertragsstaaten: **AT BE CH DE FR GB LI NL**

(71) Anmelder: **WACKER-CHEMIE GMBH**
**Prinzregentenstrasse 22**
**D-8000 München 22(DE)**

(72) Erfinder: **Meier, Josef, Dr. Dipl.-Chem.**
**Stadtplatz 95**
**D-8263 Burghausen(DE)**
Erfinder: **Deinhammer, Wolfgang, Dr. Dipl.-Ing.**
**Röntgenstrasse 32**
**D-8263 Burghausen(DE)**
Erfinder: **Krägeloh, Konrad Dr. Dipl.-Chem.**
**Marktler Strasse 3b**
**D-8263 Burghausen(DE)**

(54) **Verfahren zur Herstellung von Pyridin-2,3-dicarbonsäureestern.**

(57) Gegenstand der Erfindung ist ein Verfahren zur Herstellung von Pyridin-2,3-dicarbonsäureestern unter Verwendung neuer Maleinsäureester als Ausgangsprodukte. Bei dem Verfahren werden in einer ersten Verfahrensstufe 2,3-Dihalogenmaleinsäureester mit Ammoniak oder Ammoniumsalzen unter Ausschluß von Wasser bei Temperaturen von mindestens 50 °C unter Bildung von neuen 2-Amino-3-halogenmaleinsäureester umgesetzt und diese anschließend in einer 2. Verfahrensstufe mit α,β-ungesättigten Aldehyden oder Ketonen in Gegenwart eines säuren Katalysators und säurebindenden Mitteln bei Temperaturen von mindestens 50 °C unter Wärmezufuhr bis zur Bildung der Pyridin-2,3-dicarbonsäureester weiter umgesetzt. Derartige Pyridin-2,3-dicarbonsäureester sind beispielsweise als Zwischenprodukte für Synthesen von Herbiziden auf Imidazolinbasis verwendbar.

EP 0 371 499 A2

## Verfahren zur Herstellung von Pyridin-2,3-dicarbonsäureestern

Gegenstand der Erfindung ist ein Verfahren zur Herstellung von Pyridin-2,3-dicarbonsäureestern unter Verwendung neuer Maleinsäureester als Ausgangsprodukte. Die Pyridin-2,3-dicarbonsäureester selbst, die gegebenenfalls in 4-, 5- oder 6- Stellung substituiert sein können, sind wertvolle Zwischenprodukte für Synthesen herbizidwirksamer 2-(2-Imidazolin-2-yl)-pyridin-3-carbonsäurederivate (vgl. EP-B-41 623).

Für die Herstellung von Pyridin-2,3-dicarbonsäureestern sind folgende Verfahren bekannt:

1. Umsetzung von α,β-ungesättigten N,N,-Dialkylhydrazonen mit Halogenmaleinsäurederivaten unter Bildung von 1-Amino-1,4-dihydropyridin -2,3-dicarbonsäurederivaten und anschließende Behandlung (thermische oder Säurebehandlung), wobei unter Aminabspaltung die gewünschten Pyridinderivate entstehen (vgl. EP-A-161 221).

2. Umsetzung von α,β-ungesättigten Aldehyden oder Ketonen mit α-Halogen-β-ketoestern in Gegenwart von Ammoniumsalzen (vgl. DE-A-36 34 975) und

3. Umsetzung von β-Alkinyl-ketonen mit Aminomaleaten oder Aminofumaraten unter Bildung von ausschließlich in 6-Stellung substituierten Pyridin-2,3-dicarbonsäureestern; 5,6-Dimethylpyridin-2,3-dicarbonsäureester können hingegen durch Umsetzung von 4-Dimethyl-amino-3-methyl-butenon mit Aminofumarat erhalten werden (vgl. GB-A-21 74 395).

Verfahren 1) hat den Nachteil, daß cancerogene Hydrazinderivate eingesetzt werden und sich die Synthese über mehrere Stufen erstreckt. Bei Verfahren 2) werden nicht leicht verfügbare Halogenoxalessigester eingesetzt. Diese sind durch Claisenkondensation von Halogenessigestern und Oxalestern in mäßigen Ausbeuten herstellbar. Desgleichen werden bei dem Verfahren 3) Alkinylketone bzw. endständige Dimethylaminogruppen aufweisende Alkenylketone benötigt, die ebenfalls schwer zugänglich sind.

Es stellt sich somit die Aufgabe, ein Verfahren zur Herstellung von 2,3-Pyridin-carbonsäureestern, die in 4-, 5- und/oder 6-Stellung substituiert sein können, zur Verfügung zu stellen, das ausgehend von leicht zugänglichen Ausgangsprodukten, die beispielsweise für die Herbizidsynthese wichtigen Zwischenprodukte auf einfache und kostengünstige Weise in guten Ausbeuten liefert.

Diese Aufgabe wird erfindungsgemäß für die Herstellung von Pyridin-2,3-dicarbonsäureestern der allgemeinen Formel

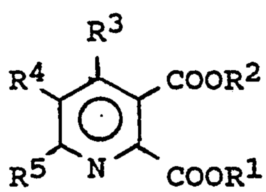

(I)

worin $R^1$ und $R^2$, die gleich oder verschieden sein können, Alkylreste mit 1 bis 8 C-Atomen und $R^3$, $R^4$ und $R^5$, die gleich oder verschieden sein können, Wasserstoffatome, Alkylreste mit 1 bis 8 C-Atomen oder Phenylreste bedeuten, dadurch gelöst, daß in einer ersten Verfahrensstufe

a) 2,3-Dihalogen-maleinsäureester der allgemeinen Formel

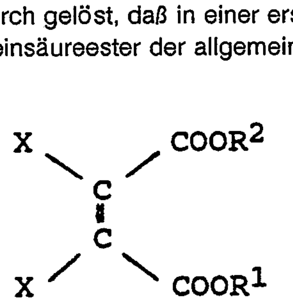

(II)

worin $R^1$ und $R^2$ die angegebene Bedeutung haben und X Halogenatome sind, mit Ammoniak oder Ammoniumsalzen in Gegenwart von organischen Lösungsmitteln unter Ausschluß von Wasser bei Temperaturen von mindestens 50 °C umgesetzt und die so gebildeten 2-Amino-3-halogen-maleinsäureester der allgemeinen Formel

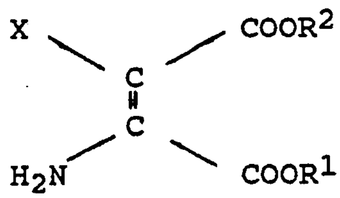

(III)

worin $R^1$, $R^2$ und X die angegebene Bedeutung haben, in einer zweiten Verfahrensstufe

b) mit α,β-ungesattigten Aldehyden oder Ketonen der allgemeinen Formel

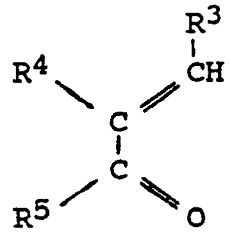

(IV)

worin $R^3$, $R^4$ und $R^5$ die angegebene Bedeutung haben, in Gegenwart eines säuren Katalysators, organischen Lösungsmitteln und säurebindenden Mitteln bei Temperaturen von mindestens 50°C unter Bildung der Verbindungen (I) umgesetzt werden.

Beispiele für Reste $R^1$ und $R^2$ in den Verbindungen der allgemeinen Formeln (I), (II) und (III) sind Alkylreste mit definitionsgemäß bis zu 8 C-Atomen, die geradkettig oder verzweigt sein können, wie Methyl-, Ethyl-, n-Propyl-, iso-Propyl-, n-Butyl-, iso-Butyl-, Pentyl-, Hexyl-, Heptyl- und Octylreste. Alkylreste mit 1 - 4 C-Atomen sind aufgrund der leichten Verfügbarkeit bevorzugt.

Beispiele für die Reste $R^3$, $R^4$ und $R^5$ in den Verbindungen der allgemeinen Formeln (I) und (IV) können außer Wasserstoffatomen und Phenylresten die gleichen Alkylreste sein, wie für $R^1$ und $R^2$ angegeben.

Beispiele für Reste X in den Verbindungen der allgemeinen Formeln (II) und (III) sind Halogenatome, vorzugsweise Chlor- oder Bromatome.

Die Verbindungen (II) und (IV) sind bekannt und können nach bekannten Verfahren hergestellt werden.

Für die Durchführung des erfindungsgemäßen Verfahrens werden in der 1. Verfahrensstufe a) die Dihalogenmaleinsäureester (II) definitionsgemäß mit Ammoniak oder Ammoniumsalzen bei Temperaturen im Bereich von 50°C bis 150°C, vorzugsweise von 60°C bis 120°C unter wasserfreien Bedingungen umgesetzt. Als Reaktionsmedien werden vorteilhaft aprotische Lösungsmittel verwendet. Beispiele hierfür sind Carbonsäureamide, wie Dimethylformamid und Dimethylacetamid. Es können jedoch auch Alkanole, wie Methanol, Ethanol, Propanol und Butanol verwendet werden. Bei Einsatz von Ammoniak wird dieses vorteilhaft in Form von trockenem gasförmigem Ammoniak so lange in die auf Reaktionstemperatur vorerhitzte Reaktionsmischung eingeleitet, bis die exotherm verlaufende Reaktion praktisch vollständig ist. Als Reaktionsmedium wird vorteilhaft Dimethylformamid verwendet, das katalytische Mengen einer Säure, vorzugsweise wasserfreie Essigsäure, enthält. Bei Einsatz von Ammoniumsalzen werden je Mol (II) mindestens 2 Mol, vorzugsweise 2,5 Mol des Ammoniumsalzes eingesetzt. Als Reaktionsmedium haben sich insbesondere Dimethylformamid oder Dimethylacetamid bewährt. Beispiele für Ammoniumsalze sind Ammoniumacetat, Ammoniumformiat und Ammoniumsulfamat, wobei Ammoniumacetat bevorzugt ist.Das Reaktionsgemisch wird auf Reaktionstemperatur vorerhitzt, wobei durch mechanische Bewegung für eine gute Verteilung der Reaktionspartner gesorgt werden kann. Die Reaktionszeit ist im allgemeinen von den eingesetzten Mengen der Ausgangsprodukte abhängig. Nach beendeter Reaktion und dem anschließenden Entfernen des Lösungsmittels werden die 2-Amino-3-halogenmaleinsäureester (III) erhalten.

Diese Verbindungen sind neu und in der Literatur noch nicht beschrieben. Sie sind bei Raumtemperatur viskose Flüssigkeiten von rötlicher Färbung oder Kristalle von gelblicher Färbung. Die so erhaltenen Verbindungen (III) können nach dem Entfernen des Lösungsmittels mit Wasser gewaschen und/oder umkristallisiert werden. Sie können aber auch direkt, das heißt ohne weitere Reinigung für die nächste Verfahrensstufe b) eingesetzt werden.

In der 2. Verfahrensstufe b) werden die Aminohalogenmaleinsäureester (III) definitionsgemäß mit α,β-ungesättigten Aldehyden oder Ketonen (IV) unter Bildung der gewünschten Pyridinderivate (I) bei Temperaturen im Bereich von 50°C bis zum Siedepunkt des Lösungsmittels, vorzugsweise von 70°C bis 110°C

umgesetzt.

Beispiele für die als Reaktionsmedien verwendbaren organischen Lösungsmittel sind Alkanole, Chlorkohlenwasserstoffe, Kohlenwasserstoffe, Ether, Carbonsäuren und deren Ester, Carbonsäurenitrile und -amide, wie Acetonitril und Dimethylformamid. Als säurebindende Mittel werden vorteilhaft Pyridin, Piccoline, N,N-Dialkylaniline, Ammonium- und Alkaliacetate eingesetzt, wobei sich Pyridin besonders bewahrt hat. Als säure Katalysatoren werden vorzugsweise Carbonsäuren, insbesondere wasserfreie Essigsäure, verwendet. Die Reaktionsteilnehmer (III) und (IV) und die säurebindenden Mittel werden in etwa äquimolaren Mengen eingesetzt, vorzugsweise wird Komponente (IV) in geringem Überschuß (1,1 Mol je Mol III) angewendet. Besonders bewährt hat sich der Einsatz von wasserfreier Essigsäure, die gleichzeitig als Lösungsmittel und Katalysator dient. Nach beendeter Umsetzung, die unter fortlaufender Wärmezufuhr im allgemeinen mehrere Stunden in Anspruch nehmen kann, werden die Pyridin-Derivate (I) mittels üblicher Techniken wie Extraktion, Eindampfen oder Säulenchromatographie isoliert.

In den folgenden Beispielen wird das erfindungsgemäße Verfahren näher erläutert.

## Beispiel 1

53,25 g (0,25 Mol) Dichlormaleinsäuredimethylester wurden in 100 ml Dimethylformamid gelöst und 48,16 g (0,625 Mol) Ammoniumacetat zugegeben. Das Gemisch wurde rasch auf 120 °C erhitzt und 1 h lang bei 110 °C bis 120 °C gerührt. Anschließend wurde das Lösungsmittel abdestilliert, der Rückstand in Methylenchlorid aufgenommen und mit Wasser gewaschen. Das Methylenchlorid wurde an einem Verdampfer abdestilliert und es wurden nach Umkristallisieren 40 g (83 % Ausbeute) Aminochlormaleinsäuredimethylester in Form gelblicher, wurfelformiger Kristalle erhalten (Fp. 96 °-98 ° C)

NMR-Spektren: $\delta$ -Werte im $CDCl_3$ :

3,85 (s, 3H, $\underline{CH_3}\text{-O-}\overset{\overset{O}{\|}}{C}$ ) 4,00

(s, 3H, $\underline{CH_3}\text{-O-}\overset{\overset{O}{\|}}{C}$ ) 5,23 (s, 2H, $NH_2$).

## Beispiel 2

48,2 g (0,2 Mol) Dichlormaleinsäurediethylester wurden in 80 ml Dimethylformamid und 2 ml Eisessig gelöst und auf 100 °C erwärmt. Dann wurde Ammoniak-Gas eingeleitet.

Die Temperatur stieg dabei auf ca. 120 °C. Nach 30 min fiel die Temperatur wieder auf 100 °C; die Reaktion war dann beendet. Anschließend wurde das Lösungsmittel im Vakuum abdestilliert. Der Rückstand wurde in Methylenchlorid aufgenommen und mit Wasser gewaschen.

Die Methylenchlorid-Lösung wurde am Rotationsverdampfer eingeengt. Man erhielt 38,9 g (87,8 % Ausbeute) Aminochlormaleinsäurediethylester als gelbes Öl, das beim Anreiben zu nadelförmigen Kristallen erstarrte (Fp. 84 ° C - 86 ° C).

$^1$H-NMR-Spektren: $\delta$-Werte in $CDCl_3$:

1,36 (t, 3H, $\underline{CH_3}\text{-}CH_2\text{-O-}\overset{\overset{O}{\|}}{C}$ );

1,42 (t, 3H, $\underline{CH_3}\text{-}CH_2O\text{-}\overset{\overset{O}{\|}}{C}$);

4,28 (q, 2H, $CH_3\text{-}\underline{CH_2}O\text{-}\overset{\overset{O}{\|}}{C}$ );

4,40 (q, 2H, $CH_3\text{-}\underline{CH_2}O\text{-}\overset{\overset{O}{\|}}{C}$ ); 5,29 (s, 2H, $-NH_2$.

## Beispiel 3

Unter gleichen Bedingungen, wie in Beispiel 1 beschrieben, wurden 26,9 g (0,1 Mol) Dichlormaleinsäuredipropylester, 19,27 g (0,25 Mol) Ammoniumacetat und 40 ml Dimethylformamid umgesetzt. Es wurden 24,1 g (87,4 % Ausbeute) Aminochlormaleinsäuredipropylester als rötliche viskose Flüssigkeit erhalten (Reinheit lt. GC-Analyse 90,5 %).

$^1$H-NMR-Spektren: $\delta$-Werte in $CDCl_3$:

0,88 (t, 3H, $CH_3$-$CH_2$-$CH_2$); 0,92 (t, 3H, $\underline{CH_3}$-$CH_2$-$CH_2$);
1,51 ($m_c$, 2H, $CH_3$-$\underline{CH_2}$-$CH_2$); 1,67 ($m_c$, 2H, $CH_3$-$\underline{CH_2}$-$CH_2$);
4,05 (t, 2H, $\overset{O}{\overset{||}{C}}$-O$\underline{CH_2}$-$CH_2$-);
4,15 (t, 2H, $\overset{O}{\overset{||}{C}}$-O$\underline{CH_2}$-$CH_2$); 5,70 (s, 2H, $NH_2$).

## Beispiel 4

Unter gleichen Bedingungen, wie in Beispiel 1 beschrieben, wurden 29,7 g (0,1 Mol) Dichlormaleinsäuredibutylester, 19,27 g (0,25 Mol) Ammoniumacetat und 40 ml Dimethylformamid umgesetzt. Es wurden 27,55 g (88,7 % Ausbeute) Aminochlormaleinsäuredibutylester als rötliche viskose Flüssigkeit erhalten (Reinheit lt. GC-Analyse 90 %).
$^1$H-NMR-Spektrum: δ-Werte im $CDCl_3$:
0,93 ($m_c$, 6H, 2x $\underline{CH_3}$-$CH_2$-$CH_2$-); 1,42 ($m_c$, 4H, 2x $CH_3$-$\underline{CH_2}$-$CH_2$-)
1,75 ($m_c$, 4H, 2x $CH_3$-$CH_2$-$\underline{CH_2}$-$CH_2$- $\overset{O}{\overset{||}{C}}$ -),
4,13 (t, 2H, $\overset{O}{\overset{||}{C}}$-O$\underline{CH_2}$-$CH_2$-)
4,25 (t, 2H, $\overset{O}{\overset{||}{C}}$-O$\underline{CH_2}$-$CH_2$-): 5,53 (s, 2H, -$NH_2$).

## Beispiel 5

Unter gleichen Bedingungen, wie in Beispiel 1 beschrieben, wurden 26,9 g (0,1 Mol) Dichlormaleinsäurediisopropylester, 19,27 g (0,25 Mol) Ammoniumacetat und 40 ml Dimethylformamid umgesetzt. Es wurden 29,65 g (85,6 % Ausbeute) Aminochlormaleinsäurediisopropylester als rötlich-gelbe viskose Flüssigkeit erhalten (Reinheit lt. GC-Analyse 83 %).
$^1$H-NMR-Spektrum: δ-Werte in $CDCl_3$:
1,31 (d, 6H, $\overset{O}{\overset{||}{C}}$-OCH($\underline{CH_3}$)$_2$);
1,39 (d, 6H, $\overset{O}{\overset{||}{C}}$-OCH($\underline{CH_3}$)$_2$;
5,16 (h, 1H $\overset{O}{\overset{||}{C}}$-O$\underline{CH}$($CH_3$)$_2$);
5,30 (h, 1H, $\overset{O}{\overset{||}{C}}$-O$\underline{CH}$($CH_3$)$_2$; 5,65 (s, 2H, $NH_2$)

## Beispiel 6

Unter gleichen Bedingungen, wie in Beispiel 1 beschrieben, wurden 29,7 g (0,1 Mol) Dichlormaleinsäurediisobutylester, 19,27 g (0,25 Mol) Ammoniumacetat und 40 ml Dimethylformamid umgesetzt; Es wurde ein rotes Öl erhalten, das beim Anreiben kristallisiert. Nach dem Waschen des Kristallbreies mit Hexan wurden 22,3 g (80 %) Aminochlormaleinsäurediisobutylester als würfelförmige Kristalle erhalten (Fp. 80 °C-81 °C).
$^1$H-NMR-Spektrum: δ-Werte in $CDCl_3$:
0,95 (d, 6H, $CH_2$-CH($CH_3$)$_2$; 0,98 (d, 6H, -$CH_2$-CH($\underline{CH_3}$)$_2$;
2,02 ($m_c$, 2H, 2x -$CH_2$-$\underline{CH}$($CH_3$)$_2$);
3,95 (d, 2H, $\overset{O}{\overset{||}{C}}$-O$\underline{CH_2}$-CH($CH_3$)$_2$);
4,05 (d, 2H, $\overset{O}{\overset{||}{C}}$-O$\underline{CH_2}$-(CH($CH_3$)$_2$); 5,02 (s, 2H, $NH_2$).

## Beispiel 7

Ein gerührtes Gemisch aus 22,16 g (0,1 Mol) Aminochlormaleinsäurediethylester, 8,92 g (0,105 Mol) Ethylacrolein, 8,3 g (0,105 Mol) Pyridin und 1 ml Eisessig in 25 ml Ethanol wurden unter Rückfluß zum Sieden erhitzt. Nach 5 h wurde das Gemisch auf Raumtemperatur abgekühlt und das Lösungsmittel unter vermindertem Druck entfernt.

Der Rückstand wurde in Methylenchlorid aufgenommen und mit Wasser gewaschen. Anschließend wurde das Lösungsmittel im Vakuum entfernt. Es wurden 25,8 g (80 % Ausbeute) 5-Ethylpyridin-2,3-dicarbonsäurediethylester als rötliches viskoses Öl erhalten. Gemäß einer Gaschromatographie-Analyse war das Produkt zu 81 % rein.

Beispiele 8 - 20

Das Verfahren von Beispiel 7 wurde unter gleichen Bedingungen wiederholt. Dabei wurden verschiedene Aminochlormaleinate der allgemeinen Formel (III) mit Ethylacrolein, Methylacrolein und Acrolein umgesetzt. Es wurden die in der folgenden Tabelle 1 angegebenen, in 5-Stellung substituierten Pyridin-2,3-dicarboxylate erhalten:

Tabelle I

| Beispiel Nr. | $R^1$ | $R^2$ | $R^4$ | Ausbeute % | Analytik |
|---|---|---|---|---|---|
| 8 | $CH_3$ | $CH_3$ | $CH_3$ | 48 | $^1H$-NMR; GC |
| 9 | $CH_3$ | $CH_3$ | $C_2H_5$ | 65 | $^1H$-NMR; GC |
| 10 | $C_2H_5$ | $C_2H_5$ | $CH_3$ | 72 | $^1H$-NMR; GC |
| 11 | $C_2H_5$ | $C_2H_5$ | H | 35 | $^1H$-NMR; GC |
| 12 | n-$C_3H_7$ | n-$C_2H_5$ | $C_2H_5$ | 70 | $^1H$-NMR; GC |
| 13 | n-$C_3H_7$ | n-$C_3H_7$ | $CH_3$ | 56 | $^1H$-NMR; GC |
| 14 | i-$C_3H_7$ | i-$C_3H_7$ | $C_2H_5$ | 81 | $^1H$-NMR; GC |
| 15 | i-$C_3H_7$ | i-$C_3H_7$ | H | 36 | $^1H$-NMR; GC |
| 16 | n-$C_4H_9$ | n-$C_4H_9$ | $C_2H_5$ | 77 | $^1H$-NMR; GC |
| 17 | n-$C_4H_9$ | n-$C_4H_9$ | H | 36 | $^1H$-NMR; GC |
| 18 | i-$C_4H_9$ | i-$C_4H_9$ | $C_2H_5$ | 68 | $^1H$-NMR; GC |
| 19 | i-$C_4H_9$ | i-$C_4H_9$ | $CH_3$ | 48 | $^1H$-NMR; GC |
| 20 | $C_2H_5$ | $C_2H_5$ | n-$C_3H_7$ | 84 | $^1H$-NMR; GC |

Die Wirksamkeit verschiedener Lösungsmittel und Basen bei der Herstellung von Diethyl-5-ethylpyridin-2,3-dicarboxylat wurde wie folgt geprüft:

Aminochlormaleinsäurediethylester wurde mit Ethylacrolein und mit verschiedenen Basen in verschiedenen Lösungsmitteln gemäß Beispiel 7 zu Diethyl-5-ethylpyridin-2,3-dicarboxylat umgesetzt. Die Ergebnisse der Experimente sind in Tabelle II zusammengefaßt. Daraus ist die Wirksamkeit der verschiedenen Basen und Lösungsmittel bei dem erfindungsgemäßen Verfahren ersichtlich.

Tabelle II

| Wirksamkeit von Basen und Lösungsmittel | | |
|---|---|---|
| Base | Lösungsmittel | Ausbeute an 5-Ethylpyridin-2,3-dicarboxylat |
| $NH_4OAc$ | EtOH *) | 37 % |
| $NEt_3$ | EtOH | 5 % |
| N,N-Dimethylanilin | EtOH | 48 % |
| NaOAc | EtOH | 9 % |
| Na-Isobutyrat | Isobuttersäure | 22 % |
| Pyridin | DMF **) | 71 % |
| Pyridin | Tri ***) | 62 % |
| Pyridin | Toluol | 58 % |
| Pyridin | Cyclohexan | 36 % |
| Pyridin | Essigester | 9 % |
| Pyridin | Acetonitril | 15 % |
| Pyridin | Diisopropylether | 16 % |
| Pyridin | Methylethylketon | 62 % |
| Pyridin | Isobuttersäure | 85 % |
| Pyridin | Isobutanol | 75 % |
| Pyridin | Aceton | 38 % |
| Pyridin | Essigsäure | 88 % |

*) EtOH = Ethanol
**) DMF = Dimethylformamid
***) Tri = Trichlorethylen

**Ansprüche**

1. Verfahren zur Herstellung von Pyridin-2,3-dicarbonsäureestern der allgemeinen Formel

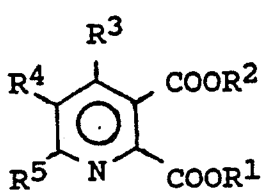

(I)

worin $R^1$ und $R^2$, die gleich oder verschieden sein können, Alkylreste mit 1 bis 8 C-Atomen und $R^3$, $R^4$ und $R^5$, die gleich oder verschieden sein können, Wasserstoffatome, Alkylreste mit 1 bis 8 C-Atomen oder Phenylreste bedeuten,
dadurch gekennzeichnet, daß in einer ersten Verfahrensstufe

a) 2,3-Dihalogenmaleinsäureester der allgemeinen Formel

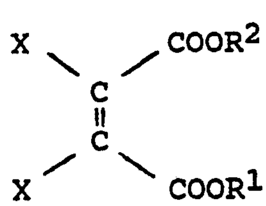

(II)

worin $R^1$ und $R^2$ die angegebene Bedeutung haben und X Halogenatome sind, mit Ammoniak oder Ammoniumsalzen in Gegenwart von organischen Lösungsmitteln unter Ausschluß von Wasser bei Temperaturen von mindestens 50 °C umgesetzt und die so gebildeten 2-Amino-3-halogen-maleinsäureester der allgemeinen Formel

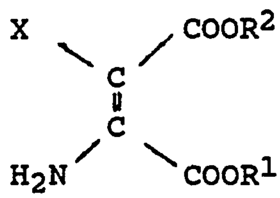

(III)

worin $R^1$, $R^2$ und X die angegebene Bedeutung haben, in einer zweiten Verfahrensstufe

b) mit α, β-ungesättigten Aldehyden oder Ketonen der allgemeinen Formel

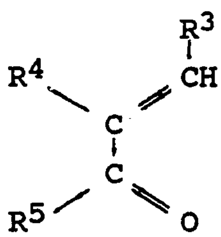

(IV)

worin $R^3$, $R^4$ und $R^5$ die angegebene Bedeutung haben, in Gegenwart eines säuren Katalysators, organischen Lösungsmitteln und säurebindenden Mitteln bei Temperaturen von mindestens 50 °C unter Bildung der Verbindungen (I) umgesetzt werden.

2. Verfahren nach Anspruch 1, dadurch gekennzeichnet, daß die Reaktion in der ersten Verfahrensstufe a) durch Einleiten von trockenem, gasförmigem Ammoniak in das vorerhitzte Reaktionsgemisch, bestehend aus dem Reaktions partner (II) und Dimethylformamid , das katalytische Mengen wasserfreier Essigsäure enthält, bei Temperaturen im Bereich von 60° bis 120 °C durchgeführt wird.

3. Verfahren nach Anspruch 1, dadurch gekennzeichnet, daß die Reaktion in der ersten Verfahrensstufe a) durch Erhitzen des Reaktionsgemisches bestehend aus den Reaktionspartnern (II) und Ammoniumacetat im Molverhältnis von 1:2,5 und Dimethylformamid bei Temperaturen im Bereich von 60 °C bis 120 °C durchgeführt wird.

4. Verfahren nach Anspruch 1, dadurch gekennzeichnet, daß die Reaktion in der zweiten Verfahrensstufe b) durch mehrstündiges Erhitzen des Reaktionsgemisches bestehend aus den Reaktionspartnern (II), (IV) und Pyridin im Molverhältnis von 1:1 bis 1,1:1 und wasserfreier Essigsäure bei Temperaturen im Bereich von 70 °C bis 110 °C durchgeführt wird.

5. 2-Amino-3-halogenmaleinsäureester der allgemeinen Formel

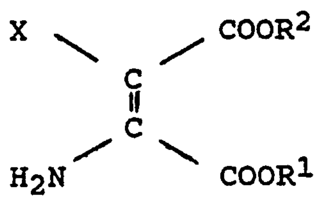

worin $R^1$ und $R^2$, die gleich oder verschieden sein können, geradkettige oder verzweigte Alkylreste mit 1 bis 8 C-Atomen und X Halogenatome sind.